# EUROPÄISCHE PATENTSCHRIFT

(11) **EP 2 146 947 B1**
(45) Veröffentlichungstag und Bekanntmachung des Hinweises auf die Patenterteilung: **12.01.2011**
(21) Anmeldenummer: 08735055.9
(22) Anmeldetag: 07.04.2008
(51) Int. Cl.: C07C 51/235, C07C 59/125

(54) **VERFAHREN ZUR HERSTELLUNG VON ALKYLPOLYGLYKOLCARBONSÄUREN UND POLYGLYKOLDICARBONSÄUREN MITTELS DIREKTOXIDATION**
METHOD FOR PRODUCING ALKYL POLYGLYCOL CARBOXYLIC ACIDS AND POLYGLYCOL DICARBOXYLIC ACIDS
PROCÉDÉ DE PRÉPARATION D'ACIDES ALKYLPOLYGLYCOLCARBOXYLIQUES ET D'ACIDES POLYGLYCOLDICARBOXYLIQUES PAR OXYDATION DIRECTE

(30) Priorität: 12.04.2007 DE 102007017179
(43) Veröffentlichungstag der Anmeldung: 27.01.2010
(73) Patentinhaber: Clariant Finance (BVI) Limited, Road Town, Tortola (VG)
(72) Erfinder: STANKOWIAK, Achim, 84503 Altötting (DE); FRANKE, Oliver, 81671 München (DE); PRUESSE, Ulf, 38118 Braunschweig (DE); DECKER, Nadine, 15537 Erkner (DE); VORLOP, Klaus-Dieter, D-38102 Braunschweig (DE)
(74) Vertreter: Mikulecky, Klaus
(86) Internationale Anmeldenummer: PCT/EP2008/002735
(87) Internationale Veröffentlichungsnummer: WO 2008/125241

(56) Entgegenhaltungen:
- EP-A- 0 653 413
- WO-A-02/16298
- US-A- 3 342 858

## Beschreibung

Alkylpolyglykolcarbonsäuren (Ethercarbonsäuren), d. h. organische Carbonsäuren, die neben der Carboxylfunktion eine oder mehrere Etherbrücken tragen, bzw. deren Alkali- oder Aminsalze, sind als milde Detergenzien mit hohem Kalkseifendispergiervermögen bekannt. Sie finden sowohl in Waschmittel- und Kosmetikformulierungen als auch in technischen Anwendungen, wie z. B. Metallbearbeitungsflüssigkeiten und Kühlschmiermittel, Verwendung.

Ethercarbonsäuren werden gemäß dem Stand der Technik entweder durch Alkylierung von Alkylpolyglykolen (Alkohol- oder Fettalkoholoxalkylaten) mit Chloressigsäurederivaten (Williamsonsche Ethersynthese) oder aus den gleichen Ausgangsprodukten durch Oxidation mit verschiedenen Reagenzien (Luftsauerstoff, Hypochlorit, Chlorit) unter Katalyse mit verschiedenen Katalysatoren dargestellt. Die Williamsonsche Ethersynthese stellt vor allem aufgrund der Kosten-Wirkung-Beziehung das technisch geläufigste Verfahren für die Herstellung von Ethercarbonsäuren dar, jedoch besitzen durch dieses Verfahren hergestellte Produkte noch gravierende Mängel in Bezug auf die Handhabbarkeit für den Anwender, wie beispielsweise Löslichkeitsverhalten, Aggregatzustand bei niedrigen Temperaturen und Lagerstabilität.

Diese Mängel sind im Wesentlichen auf verfahrensbedingte Nebenbestandteile zurückzuführen. So werden trotz Verwendung von Überschüssen des entsprechenden Chloressigsäurederivats nur Umsätze von ca. 70-85 % erreicht, so dass Restmengen von Oxethylat und Fettalkohol, der dem Oxethylat zugrunde liegt, im Endprodukt verbleiben. Des Weiteren entstehen durch den zu verwendenden Überschuss des Chloressigsäurederivats Folgeprodukte, wie beispielsweise Glykolsäure, Diglykolsäure und deren Derivate, die eine wesentliche Ursache für die Alterung der Produkte sind und gegebenenfalls Probleme beim Löslichkeitsverhalten hervorrufen können.

Ein weiterer Nachteil der Williamsonschen Synthese besteht in der hohen Belastung der Reaktionsprodukte durch Natriumchlorid, das in wässrigen Lösungen eine wesentliche Ursache für Lochfraß-Korrosion darstellt. Außerdem gelangt das gebildete Natriumchlorid in das Reaktionsabwasser und stellt dort für biologische Kläranlagen ein Problem dar, da Kochsalz die Reinigungsleistung solcher Anlagen beeinträchtigen kann.

Die direkte Oxidation von Alkoholoxethylaten zu Ethercarbonsäuren gelingt mithilfe von Platin-Katalysatoren, wie z. B. in US-3 342 858 beschrieben. Platin kann sowohl als Suspension verwendet werden, als auch auf einem Trägermaterial wie Kohlenstoff aufgebracht sein. Die Oxidation wird in alkalischer Lösung bei einer Temperatur von 20 bis 75 °C und einem maximalen Druck von 3 bar durchgeführt. Nachteil dieses Verfahrens sind die sehr verdünnten Lösungen (3 bis 12 %ige wässrige Lösungen), die teilweise langen Reaktionszeiten von bis zu 24 Stunden und die damit verbundene geringe Raum-Zeit-Ausbeute. Nachteilig sind bei den verwendeten Platin-Katalysatoren ebenfalls die geringen Selektivitäten; die Ausbeuten betragen nach destillativer Aufarbeitung nur ca. 68 bis 89 %.

Überraschend wurde nun gefunden, dass Ethercarbonsäuren und deren Salze sowie Polyglykoldicarbonsäuren und deren Salze auch durch direkte Oxidation von Alkylpolyglykolen bzw. Polyglykolen mit Luftsauerstoff oder Reinsauerstoff mittels goldhaltigen Katalysatoren in hoher Ausbeute zugänglich sind.

Gegenstand der vorliegenden Erfindung ist daher ein Verfahren zur Herstellung von Verbindungen der Formel (Ia) und/oder Verbindungen der Formel (Ib) 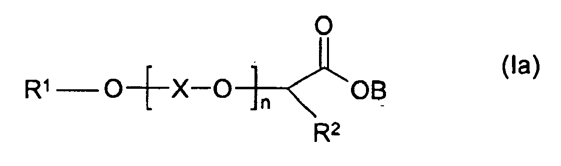 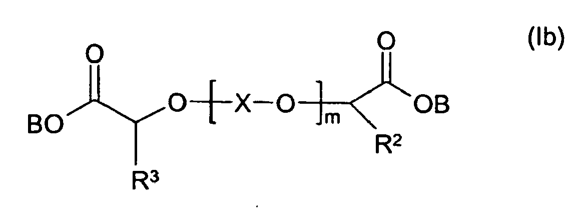 worin
- R¹: einen gesättigten, linearen oder verzweigten Alkylrest mit 1 bis 22 Kohlenstoffatomen oder einen ein- oder mehrfach ungesättigten linearen oder verzweigten Alkenylrest mit 2 bis 22 Kohlenstoffatomen,
- R², R³: unabhängig voneinander Wasserstoff, einen linearen oder verzweigten Alkylrest mit 1 bis 22 Kohlenstoffatomen, einen ein- oder mehrfach ungesättigten linearen oder verzweigten Alkenylrest mit 2 bis 22 Kohlenstoffatomen, oder einen Arylrest mit 6 bis 12 Kohlenstoffatomen,
- X: ein Alkylenrest mit 2 bis 4 Kohlenstoffatomen,
- n: eine Zahl zwischen 0 und 100,
- m: eine Zahl zwischen 1 und 250, und
- B: ein Kation oder Wasserstoff
bedeuten, und/oder der entsprechenden protonierten Carbonsäuren, indem ein oder mehrere Verbindungen der Formel (IIa) und/oder der Formel (IIb) 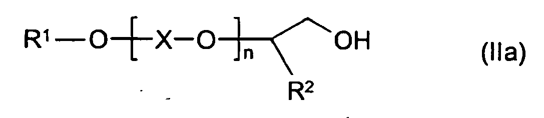 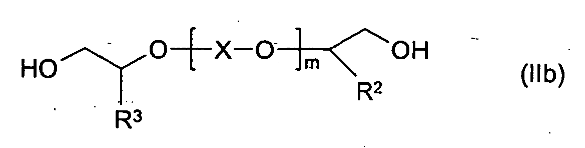 worin R¹, R², R³, X, n und m die oben angegebene Bedeutung besitzen, mit Sauerstoff oder Sauerstoff enthaltenden Gasen in Gegenwart eines goldhaltigen Katalysators und mindestens einer alkalischen Verbindung oxidiert werden.

Bevorzugt ist R¹ ein linearer oder verzweigter Alkylrest mit 1 bis 12 Kohlenstoffatomen oder ein ein- oder mehrfach ungesättigter, linearer oder verzweigter Alkenylrest mit 2 bis 12 Kohlenstoffatomen. Besonders bevorzugt sind Methyl, Butyl und Lauryl. R¹ ist vorzugsweise gesättigt.

Bevorzugt sind R² und R³ unabhängig voneinander Wasserstoff oder ein C₁ bis C₄- Alkylrest

Bei der Polyglykolkette (X-O) der Ausgangsverbindungen (IIa) und (IIb) kann es sich um eine reine oder gemischte Alkylenoxid-Kette mit statistischer oder blockweiser Verteilung von (X-O)-Gruppen handeln.

Als alkalische Verbindung können Carbonate, Hydroxide oder Oxide in dem erfindungsgemäßen Verfahren verwendet werden. Bevorzugt sind die Hydroxide BOH.

Bei den Gegenionen B handelt es sich vorzugsweise um Alkalimetallkationen, ausgewählt aus Kationen der Alkalimetalle Li, Na, K, Rb und Cs. Besonders bevorzugt sind die Kationen der Alkalimetalle Na und K. Als alkalische Verbindung im erfindungsgemäßen Verfahren sind die Hydroxide von Li, Na, K, Rb und Cs besonders bevorzugt.

Der goldhaltige Katalysator kann ein reiner Goldkatalysator oder ein gemischter Katalysator sein, der neben Gold weitere Metalle der Gruppe VIII enthält. Bevorzugt sind als Katalysatoren Goldkatalysatoren, die zusätzlich mit einem der Metalle aus der Gruppe VIII dotiert sind. Besonders bevorzugt ist die Dotierung mit Platin oder Palladium.

Vorzugsweise sind die Metalle auf Trägern aufgebracht. Bevorzugte Träger sind Aktivkohle und oxidische Träger, vorzugsweise Titandioxid, Cerdioxid oder Aluminiumoxid. Solche Katalysatoren können nach den bekannten Methoden wie Incipient Wetness (IW) oder Deposition-Precipitation (DP) wie z. B. in L. Prati, G. Martra, Gold Bull. 39 (1999) 96 und S. Biella, G.L. Castiglioni, C. Fumagalli, L. Prati, M. Rossi, Catalysis Today 72 (2002) 43-49 oder L. Prati, F. Porta, Applied catalysis A: General 291 (2005) 199-203 beschrieben, hergestellt werden.

Die geträgerten reinen Goldkatalysatoren enthalten vorzugsweise 0,1 bis 5 Gew.-% Gold, bezogen auf das Gewicht des Katalysators, der aus Träger und Gold besteht.

Enthält der Katalysator Gold und ein weiteres Metall, so handelt es sich vorzugsweise um 0,1 bis 5 Gew.-% Gold und 0,1 bis 3 Gew.-% eines Gruppe VIII-Metalls, bevorzugt Platin oder Palladium. Besonders bevorzugt sind solche Katalysatoren, die 0,5 bis 3 Gew.-% Gold enthalten. Das bevorzugte Gewichtsverhältnis Gold/Gruppe VIII Metall, insbesondere Gold/Platin oder Gold/Palladium, beträgt 70 : 30 bis 95 : 5.

In einer weiteren bevorzugten Ausführungsform ist der reine Goldkatalysator ein Nanogoldkatalysator mit einer Teilchengröße von vorzugsweise 1 bis 50 nm, besonders bevorzugt 2 bis 10 nm. Reine Nanogoldkatalysatoren enthalten vorzugsweise 0,1 bis 5 Gew.-% Gold, besonders bevorzugt sind 0,5 bis 3 Gew.-% Gold. Enthält der Katalysator Nanogold und ein weiteres Metall, so handelt es sich vorzugsweise um 0,1 bis 5 Gew.-% Nanogold und 0,1 bis 2 Gew.-% eines Gruppe VIII-Metalls, bevorzugt Platin öder Palladium. Besonders bevorzugt sind solche Katalysatoren, die 0,5 bis 3 Gew.-% Nanogold enthalten. Das bevorzugte Gewichtsverhältnis Nanogold/Gruppe VIII Metall, insbesondere Nanogold/Platin oder Nanogold/Palladium, beträgt 70 : 30 bis 95 : 5.

Das erfindungsgemäße Verfahren wird vorzugsweise in Wasser durchgeführt.

Die Oxidationsreaktion wird bei einer Temperatur von 30 bis 200 °C, bevorzugt zwischen 80 und 150 °C durchgeführt.

Der pH-Wert während der Oxidation liegt bevorzugt zwischen 8 und 13, besonders bevorzugt zwischen 9 und 11.

Der Druck bei der Oxidationsreaktion ist vorzugsweise im Vergleich zu Atmosphärendruck erhöht.

Bei der Reaktion im alkalischen Medium entstehen zunächst die Alkalisalze (B = Li, Na, K, Rb, Cs) der Carbonsäuren, bevorzugt die Natrium- oder Kaliumsalze. Zur Herstellung der freien Ethercarbonsäure (d. h. B = Wasserstoff) werden die erhaltenen Ethercarboxylate der Formel (la) oder (Ib) mit Säuren umgesetzt. Bevorzugte Säuren sind Salz- und Schwefelsäure.

Das erfindungsgemäße Verfahren ergibt vorzugsweise Lösungen von Carboxylaten der Formel (la) und/oder der Formel (Ib) mit nur noch geringen Restgehalten an Alkylpolyglykolen (IIa) und/oder Polyglykolen (IIb) von < 10 Gew.-%, bevorzugt < 5 Gew.-%, besonders bevorzugt < 2 Gew.-%.

### Beispiele

### Beispiel 1: Verfahren zur Herstellung von Ethercarboxylaten unter Verwendung von Goldkatalysatoren

In einen 2-Liter-Druckautoklaven mit Begasungsrührer werden 1 Liter einer 50 gew.-%igen Methylpolyethylenglykol (M_{W} = 1.000 g/mol) wässrigen Lösung gegeben. Nach Zugabe von 10 g eines Nanogoldkatalysators (2,5 Gew.-% Gold auf Aluminiumoxid, Teilchengröße 4 bis 8 nm) wird die Suspension mit Natronlauge auf pH 10 eingestellt und auf 100 °C aufgeheizt. Nach Erreichen der Reaktionstemperatur wird die Reaktionslösung mit Sauerstoff auf einen Druck von 8 bar aufgepresst und durch Nachpressen auf diesem Druck gehalten. Während der gesamten Reaktionszeit wird mittels eines Autotitrators der pH-Wert der Mischung mit Natronlauge auf 10 gehalten. Nach 8 Stunden wird der Reaktor abgekühlt, entspannt und der Katalysator durch Filtration von der Reaktionslösung abgetrennt. Die Lösung zeigt einen Gehalt von ca. 50 Gew.-% Methylpolyethylenglykolcarboxylat, Methylpolyethylenglykol ist nicht mehr nachweisbar.

### Beispiel 2: Verfahren zur Herstellung von Ethercarboxylaten unter Verwendung von Goldkatalysatoren

In einen 2-Liter-Druckautoklaven mit Begasungsrührer werden 1 Liter einer 20 gew.-%igen Laurylpolyglykol (M_{W} = 1.000 g/mol) wässrigen Lösung gegeben. Nach Zugabe von 6 g eines Goldkatalysators (0,9 Gew.-% Gold und 0,1 Gew.-% Platin auf Titandioxid, Teilchengröße 4 bis 8 nm) wird die Suspension mit Natronlauge auf pH 11 eingestellt und auf 80 °C aufgeheizt. Nach Erreichen der Reaktionstemperatur wird die Reaktionslösung mit Sauerstoff auf einen Druck von 8 bar aufgepresst und durch Nachpressen auf diesem Druck gehalten. Während der gesamten Reaktionszeit wird mittels eines Autotitrators der pH-Wert der Mischung mit Natronlauge auf 11 gehalten. Nach 4 Stunden wird der Reaktor abgekühlt, entspannt und der Katalysator durch Filtration von der Reaktionslösung abgetrennt. Die Lösung zeigt einen Gehalt von ca. 20 Gew.-% Laurylpolyglykolcarboxylat, Laurylpolyglykol ist nicht mehr nachweisbar.

### Beispiel 3: Verfahren zur Herstellung von Polyglykoldicarboxylaten unter Verwendung von Goldkatalysatoren

In einen 2-Liter-Druckautoklaven mit Begasungsrührer werden 1 Liter einer 50 gew.-%igen Polyethylenglykol (M_{W} = 2.000 g/mol) wässrigen Lösung gegeben. Nach Zugabe von 9 g eines Goldkatalysators (0,9 Gew.-% Gold und 0,1 Gew.-% Platin auf Titandioxid, Teilchengröße 4 bis 8 nm) wird die Suspension mit - Natronlauge auf pH 10 eingestellt und auf 80 °C aufgeheizt. Nach Erreichen der Reaktionstemperatur wird die Reaktionslösung mit Sauerstoff auf einen Druck von 10 bar aufgepresst und durch Nachpressen auf diesem Druck gehalten. Während der gesamten Reaktionszeit wird mittels eines Autotitrators der pH-Wert der Mischung mit Natronlauge auf 10 gehalten. Nach 6 Stunden wird der Reaktor abgekühlt, entspannt und der Katalysator durch Filtration von der Reaktionslösung abgetrennt. Die Lösung zeigt einen Gehalt von ca. 50 Gew.-% Polyethylenglykoldicarboxylat, Polyethylenglykol ist nicht mehr nachweisbar.

## Patentansprüche

1. Verfahren zur Herstellung von Verbindungen der Formel (la) und/oder Verbindungen der Formel (Ib) 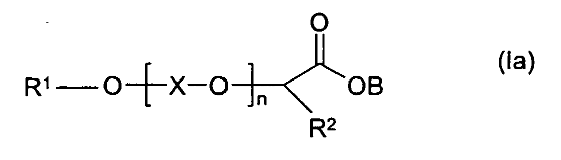 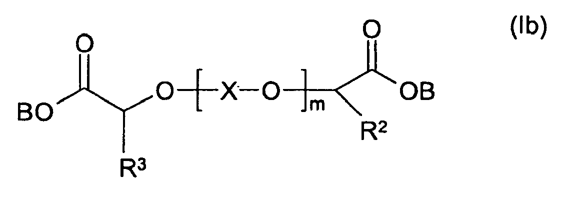 worin
R¹ einen gesättigten, linearen oder verzweigten Alkylrest mit 1 bis 22 Kohlenstoffatomen oder einen ein- oder mehrfach ungesättigten linearen oder verzweigten Alkenylrest mit 2 bis 22 Kohlenstoffatomen,
R², R³ unabhängig voneinander Wasserstoff, einen linearen oder verzweigten Alkylrest mit 1 bis 22 Kohlenstoffatomen, einen ein- oder mehrfach ungesättigten linearen oder verzweigten Alkenylrest mit 2 bis 22 Kohlenstoffatomen, oder einen Arylrest mit 6 bis 12 Kohlenstoffatomen,
X ein Alkylenrest mit 2 bis 4 Kohlenstoffatomen,
n eine Zahl zwischen 0 und 100,
m eine Zahl zwischen 1 und 250, und
B ein Kation oder Wasserstoff
bedeuten, und/oder der entsprechenden protonierten Carbonsäuren, indem ein oder mehrere Verbindungen der Formel (IIa) und/oder der Formel (IIb) 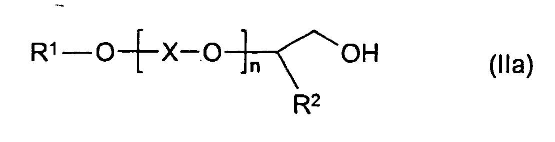 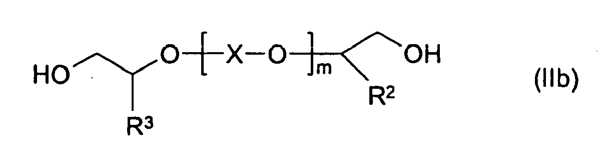 worin R¹, R², R³, X, n und m die oben angegebene Bedeutung besitzen, mit Sauerstoff oder Sauerstoff enthaltenden Gasen in Gegenwart eines goldhaltigen Katalysators und mindestens einer alkalischen Verbindung oxidiert werden.

2. Verfahren nach Anspruch 1, **dadurch gekennzeichnet, dass** der goldhaltige Katalysator ein Nanogold-Katalysator mit einer mittleren Teilchengröße von 1 bis 50 nm ist.

3. Verfahren nach Anspruch 2, **dadurch gekennzeichnet, dass** der Nanogold-Katalysator auf einem oxidischen Träger oder auf Kohlenstoff aufgebracht ist.

4. Verfahren nach Anspruch 3, **dadurch gekennzeichnet, dass** der oxidische Träger aus Titandioxid, Aluminumoxid oder Cerdioxid besteht.

5. Verfahren nach einem oder mehreren der Ansprüche 2 bis 4, **dadurch gekennzeichnet, dass** der Nano-Gold-Katalysator 0,1 bis 5 Gew.-% Nanogold enthält.

6. Verfahren nach einem oder mehreren der Ansprüche 2 bis 5, **dadurch gekennzeichnet, dass** der Nano-Gold-Katalysator 0,1 bis 5 Gew.-% Nanogold und 0,1 bis 2 Gew.-% eines Gruppe VIII Metalls enthält.

7. Verfahren nach einem oder mehreren der Ansprüche 2 bis 6, **dadurch gekennzeichnet, dass** der goldhaltige Katalysator Gold und ein weiteres Element der Gruppe VIII im Gewichtsverhältnis Au : Gruppe VIII-Metall = 70 : 30 bis 95 : 5 enthält.

8. Verfahren nach einem oder mehreren der Ansprüche 2 bis 7, **dadurch gekennzeichnet, dass** R¹ ein linearer oder verzweigter Alkylrest mit 1 bis 12 Kohlenstoffatomen oder ein ein- oder mehrfach ungesättigter, linearer oder verzweigter Alkenylrest mit 2 bis 12 Kohlenstoffatomen ist.

9. Verfahren nach einem oder mehreren der Ansprüche 2 bis 8, **dadurch gekennzeichnet, dass** R² und R³ unabhängig voneinander Wasserstoff oder ein C₁ bis C₄- Alkylrest sind.

10. Verfahren nach einem oder mehreren der Ansprüche 2 bis 9, **dadurch gekennzeichnet, dass** B Wasserstoff oder ein Kation der Alkalimetalle Li, Na, K, Rb und Cs ist.

## Claims

1. A method for producing compounds of the formula (Ia) and/or compounds of the formula (Ib) 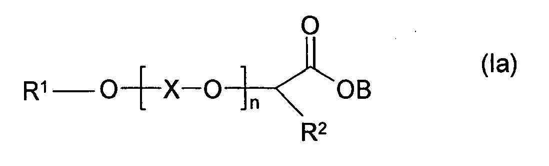 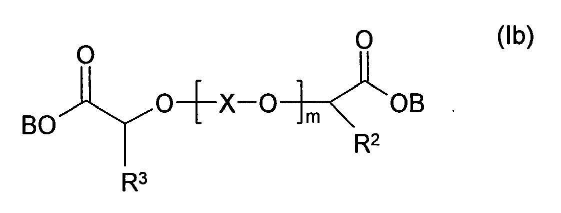 in which
R¹ is a saturated, linear or branched alkyl radical having 1 to 22 carbon atoms or a mono- or polyunsaturated linear or branched alkenyl radical having 2 to 22 carbon atoms,
R², R³ independently of one another are hydrogen, a linear or branched alkyl radical having 1 to 22 carbon atoms, a mono- or polyunsaturated linear or branched alkenyl radical having 2 to 22 carbon atoms, or an aryl radical having 6 to 12 carbon atoms,
X is an alkylene radical having 2 to 4 carbon atoms,
n is a number between 0 and 100,
m is a number between 1 and 250, and
B is a cation or hydrogen,
and/or of the corresponding protonated carboxylic acids by oxidizing one or more compounds of the formula (IIa) and/or of the formula (IIb) 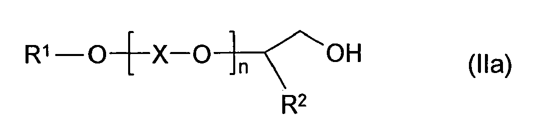 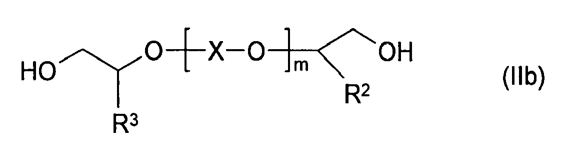 in which R¹, R², R³, X, n and m have the meaning given above, with oxygen or gases containing oxygen in the presence of a gold-containing catalyst and at least one alkaline compound.

2. The method as claimed in claim 1, wherein the gold-containing catalyst is a nanogold catalyst with an average particle size of from 1 to 50 nm.

3. The method as claimed in claim 2, wherein the nanogold catalyst is applied to an oxidic support or to carbon.

4. The method as claimed in claim 3, wherein the oxidic support comprises titanium dioxide, aluminum oxide or cerium dioxide.

5. The method as claimed in one or more of claims 2 to 4, wherein the nanogold catalyst comprises 0.1 to 5% by weight of nanogold.

6. The method as claimed in one or more of claims 2 to 5, wherein the nanogold catalyst comprises 0.1 to 5% by weight of nanogold and 0.1 to 2% by weight of a group VIII metal.

7. The method as claimed in one or more of claims 2 to 6, wherein the gold-containing catalyst comprises gold and a further element of group VIII in the weight ratio Au:group VIII metal = 70:30 to 95:5.

8. The method as claimed in one or more of claims 2 to 7, wherein R¹ is a linear or branched alkyl radical having 1 to 12 carbon atoms or a mono- or polyunsaturated, linear or branched alkenyl radical having 2 to 12 carbon atoms.

9. The method as claimed in one or more of claims 2 to 8, wherein R² and R³, independently of one another, are hydrogen or a C₁ to C₄-alkyl radical.

10. The method as claimed in one or more of claims 2 to 9, wherein B is hydrogen or a cation of the alkali metals Li, Na, K, Rb and Cs.

## Revendications

1. Procédé pour la préparation de composés de formule (Ia) et/ou de composés de formule (Ib) 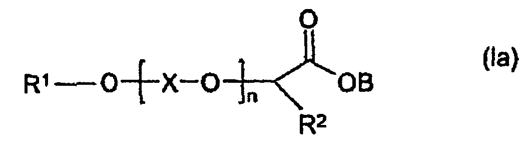 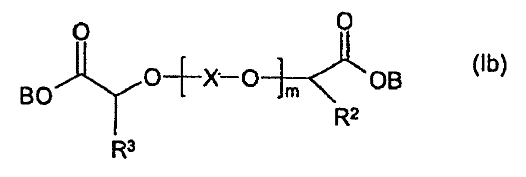 où
R¹ signifie un radical alkyle saturé, linéaire ou ramifié, comprenant 1 à 22 atomes de carbone ou un radical alcényle monoinsaturé ou polyinsaturé, linéaire ou ramifié comprenant 2 à 22 atomes de carbone,
R², R³ signifient, indépendamment l'un de l'autre, hydrogène, un radical alkyle linéaire ou ramifié, comprenant 1 à 22 atomes de carbone, un radical alcényle monoinsaturé ou polyinsaturé, linéaire ou ramifié comprenant 2 à 22 atomes de carbone ou un radical aryle comprenant 6 à 12 atomes de carbone,
X signifie un radical alkylène comprenant 2 à 4 atomes de carbone,
n vaut un nombre entre 0 et 100,
m vaut un nombre entre 1 et 250, et
B signifie un cation ou hydrogène
et/ou des acides carboxyliques protonés correspondants, en ce qu'un ou plusieurs composés de formule (IIa) et/ou de formule (IIb) 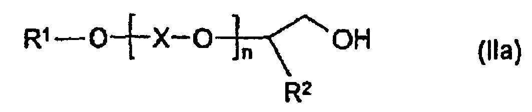 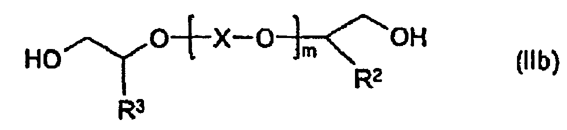 où R¹, R², R³, X, n et m présentent la signification indiquée ci-dessus, sont oxydés avec de l'oxygène ou des gaz contenant de l'oxygène en présence d'un catalyseur contenant de l'or et d'au moins un composé alcalin.

2. Procédé selon la revendication 1, **caractérisé en ce que** le catalyseur contenant de l'or est un catalyseur à base d'or-nano présentant une grosseur moyenne des particules de 1 à 50 nm.

3. Procédé selon la revendication 2, **caractérisé en ce que** le catalyseur à base d'or-nano est appliqué sur un support oxyde ou sur du carbone.

4. Procédé selon la revendication 3, **caractérisé en ce que** le support oxyde est constitué de dioxyde de titane, d'oxyde d'aluminium ou de dioxyde de cérium.

5. Procédé selon l'une ou plusieurs des revendications 2 à 4, **caractérisé en ce que** le catalyseur à base d'ornano contient 0,1 à 5% en poids d'or-nano.

6. Procédé selon l'une ou plusieurs des revendications 2 à 5, **caractérisé en ce que** le catalyseur à base d'ornano contient 0,1 à 5% en poids d'or-nano et 0,1 à 2% en poids d'un métal du groupe VIII.

7. Procédé selon l'une ou plusieurs des revendications 2 à 6, **caractérisé en ce que** le catalyseur contenant de l'or contient de l'or et un autre élément du groupe VIII dans un rapport pondéral Au:métal du groupe VIII = 70:30 à 95:5.

8. Procédé selon l'une ou plusieurs des revendications 2 à 7, **caractérisé en ce que** R¹ représente un radical alkyle linéaire ou ramifié, comprenant 1 à 12 atomes de carbone ou un radical alcényle monoinsaturé ou polyinsaturé, linéaire ou ramifié comprenant 2 à 12 atomes de carbone.

9. Procédé selon l'une ou plusieurs des revendications 2 à 8, **caractérisé en ce que** R² et R³ représentent indépendamment l'un de l'autre, hydrogène ou un radical C₁-C₄-alkyle.

10. Procédé selon l'une ou plusieurs des revendications 2 à 9, **caractérisé en ce que** B représente hydrogène ou un cation des métaux alcalins Li, Na, K, Rb et Cs.
